(19) Europäisches Patentamt
European Patent Office
Office européen des brevets

(11) Publication number: 0 001 907
B1

(12) # EUROPEAN PATENT SPECIFICATION

(21) Application number: 78300555.6

(22) Date of filing: 27.10.78

(51) Int. Cl.³: A 61 K 9/20, A 61 K 31/445

(54) ## Anaesthetic lozenges and method of preparing them.

(30) Priority: 07.11.77 US 848819

(43) Date of publication of application: 16.05.79 Bulletin 79/10

(45) Publication of the grant of the European patent: 11.02.81 Bulletin 81/6

(84) Designated Contracting States: BE DE FR GB NL SE

(56) References cited:
US - A - 2 771 391
US - A - 2 868 689
US - A - 3 439 089
US - A - 3 511 914

(73) Proprietor: BEECHAM INC.
65 Industrial South
Clifton New Jersey 07012 (US)

(72) Inventor: Lane, Philip A.
555 84th Street
Brooklyn New York 11209 (US)

(72) Inventor: Brown, Bruce A.
298 Lafayette Avenue
Brooklyn New York 11238 (US)

(74) Representative: Russell, Brian John et al,
European Patent Attorney Beecham
Pharmaceuticals Yew Tree Bottom Road
Epsom Surrey, KT18 5XQ (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 001 907 B1

Courier Press, Leamington Spa, England.

Anesthetic lozenges and method of preparing them

The present invention is concerned with anesthetic lozenges.

3-piperidino-*p*-alkoxypropiophenones wherein the alkoxy group contains from 2 to 8 carbon atoms and in particular the acid addition salts of these compounds are a known class of local anesthetic agents (see e.g. U.S. Patent No. 2,771,391 and Profft, Chem. Tech. (Berlin) *4*, 241 (1952)). Two particularly useful members of this class are dyclonine hydrochloride which is 3-piperidino-4'-butoxypropiophenone hydrochloride and falicain which is 3-piperdino-4'-oropoxypropiophenone hydrochloride. These compounds when topically applied, as for example in a 0.5% solution or a 1% cream, produce topical or local anesthetic effects. It is also known, however, that these compounds lack thermal stability, particularly in aqueous media and the use of chlorobutanol has been proposed as a stabilizer for aqueous pharmaceutical preparations of dyclonine hydrochloride (see U.S. Patent No. 2,868,689). It appears the same stability problems attach to falicain since the Merck Index indicates that aqueous solutions of this topical anesthetic should not be sterilized by autoclave methods.

Attempts to prepare a lozenge or trouche in which such compounds are mixed with a hard candy base demonstrated the existence of further thermal stability problems. Hence while a compound such as dyclonine hydrochloride is stable up to about 150°C (its melting point being 178°C), a mixture with a conventional hard candy base results in degradation at temperatures as low as 120°C. In view of the documented lack of stability of this compound in aqueous media, it was postulated that a similar phenomenon occurred in the case of dyclonine hydrochloride and the molten hard candy base. Analytical studies employing UV and IR spectroscopy and thin layer chromatography demonstrated that the decomposition product formed in aqueous solutions of dyclonine hydrochloride was also present in lozenges.

More particularly, the present invention is based on the discovery that the degradation which occurs upon mixing a 3-piperidino-*p*-alkoxypropiophenone salt with a molten hard candy base can be minimized or eliminated through either the prior or simultaneous addition of an amount of a pharmaceutically acceptable acid to the molten hard candy base sufficient to stabilize thermally said salt.

Accordingly, the present invention provides a method of preparing topical anaesthetic lozenges containing a pharmaceutically acceptable acid addition salt of a compound of formula (I)

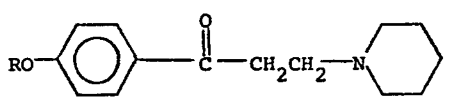

(I) .

wherein R is alkyl of 2 to 8 carbon atoms by incorporating said acid addition salt in a molten candy base and moulding into lozenges, characterised in that citric acid, fumaric acid, malic acid, tartaric acid, lactic acid, adipic acid or phosphoric acid is added to the molten candy base prior to or simultaneously with the addition of said acid addition salt, in an amount to constitute from 0.2% to 1% by weight of the resultant lozenges. Preferably, citric acid is added in an amount to constitute from 0.25% to 0.5% of the resultant lozenge. The preferred acid addition salt is the hydrochloride salt and the preferred compound of formula (I) is where R is n-butyl.

The effect of citric acid on the thermal decomposition of dyclonine hydrochloride in molten hard candy base can be seen from the following:

TABLE 1

| Temperature | Dyclonine HCl | Candy Base | Citric Acid | % Recovery |
|---|---|---|---|---|
| 85°C | + | - | - | 104 |
| 85°C | + | + | - | 100 |
| 100°C | + | - | - | 104 |
| 100°C | + | + | - | 98 |
| 100°C | + | + | + | 103 |
| 105°C | + | + | - | 98 |
| 110°C | + | + | - | 91 |
| 120°C | + | - | - | 102 |
| 120°C | + | + | - | 69 |
| 120°C | + | + | - | 87 |
| 125°C | + | + | - | 71 |
| 125°C | + | + | + | 94 |
| 135°C | + | - | - | 99 |
| 135°C | + | + | - | 55 |
| 150°C | + | - | - | 102 |
| 150°C | + | + | - | 20 |
| 185°C * | + | - | - | 20 |

* Above melting point 178°C.

As can be seen from the above, dyclonine hydrochloride is itself stable up to about 150°C but decomposes in the presence of molten candy base at about 120°C. The presence of citric acid, however, stabilizes the dyclonine hydrochloride at 125°C.

It appears that as little as 0.25% of citric acid will stabilize the dyclonine hydrochloride and molten candy base although superior stabilization is achieved utilizing levels of 0.5% citric acid. No significant increase in stabilization appears above 1% and therefore the amount of acid added in excess of this lower limit is not critical and is determined solely by economic considerations. The effect of various amounts of citric acid can be seen from Table 2 in which the recovery values of dyclonine hydrochloride which has been added to molten candy base at 135°C is shown.

TABLE 2

| % Citric Acid | % Recovery |
|---|---|
| – | 62 |
| 0.25 | 86 |
| 0.50 | 96 |
| 0.75 | 93 |
| 1.0 | 96 |

Within the range of 105°C to 135°C, which corresponds to temperatures encountered in actual process conditions, the effect of 1% citric acid on the recovery of dyclonine hydrochloride from a molten candy base can be seen in Table 3.

TABLE 3

| Temperature | Citric Acid (1%) | % Recovery |
|---|---|---|
| 105°C | No | 100 |
| 105°C | Yes | 96 |
| 115°C | No | 97 |
| 115°C | Yes | 100 |
| 125°C | No | 85 |
| 125°C | Yes | 97 |
| 135°C | No | 64 |
| 135°C | Yes | 96 |

In a large scale evaluation, four lots of dyclonine hydrochloride lozenges were prepared, one lot of which contained no citric acid and the remaining three of which contained 0.85% citric acid. For the three lots which did contain citric acid, the average percent recoveries were 105% (24 batches), 91.1% (22 batches), and 91.9% (19 batches). The average recovery for the lot which contained no citric acid was 75.2% (22 batches).

As noted above, the acid can be added to the molten candy mass prior to adding the anesthetic compound or can be intimately mixed with the active ingredient to form a homogeneous powder which is then added to the molten candy mass. Generally, the amount of topical anesthetic compound added will be such as to provide from about 0.025 to 1% by weight of the final lozenge. Preferably, the amount of topical anesthetic compound is 0.025—0.1%. A particularly preferred amount is 0.05%. The bulk of the lozenge will be composed of the hard candy base, serving as a carrier and of a conventional composition in the pharmaceutical art. In addition, flavouring agents such as methanol or methylsalicylate can be added, together with pharmaceutically acceptable colours.

In a typical embodiment, the molten candy base is prepared in the conventional manner and transferred from the cooker to a transfer pan. The acid, for example citric acid, is added to the base prior to the addition of the dyclonine hydrochloride or simultaneously as a powdered mix. Any flavouring is added and the entire mixture is well blended. The candy mass is then transferred to a kneading table, mixed and lozenges formed.

Although 3-piperidino-*p*-n-butoxypropiophenone is the preferred topical anesthetic agent, the present invention is equally useful for other compounds of this class such as 3-piperidino-*p*-n-octyloxypropiophenone, 3-piperidino-*p*-n-ethoxypropiophenone; 3-piperidino-*p*-n-amoxypropiophenone, 3-piperidino-*p*-isobutoxypropiophenone, 3-piperidino-p-sec.-butoxypropiophenone, 3-piperidino-*p*-n-propoxypropiophenone, 3-piperidino-*p*-isoamoxypropiophenone, 3-piperidino-*p*-isopropoxypropiophenone, 3-piperidino-*p*-n-heptyloxypropiophenone and 3-piperidino-*p*-n-hexyloxypropiophenone.

Likewise, although the hydrochloride salt is preferred since it is readily available, other pharmaceutically acceptable salts of these piperidino compounds and falicain such as those formed with hydrobromic acid, fumaric acid, malic acid, tartaric acid, lactic acid, adipic acid, phosphoric acid, sulfuric acid, methane sulfonic acid, naphthene disulphonic and acetic acid may also be utilized in a similar manner.

The resultant lozenge provides relief of pain and discomfort due to minor sore throats, minor mouth irritations, stomatitis and the pain and discomfort following periodontal procedures and minor oral surgery as a result of its anesthetic properties.

The following non-limitative examples more particularly illustrate the present invention:

EXAMPLE 1

| Ingredient | Amount/Lozenge |
|---|---|
| 3-Piperidino-p-butoxypropiophenone HCl | 0.60 mg |
| Anhydrous Citric Acid | 20.40 mg |
| Flavour | 12.42 mg |
| Colouring Agent | 0.222 mg |
| Anhydrous Candy Base to | QS 2.40 gm |

The candy base is melted in a vacuum cooker and the colouring agent is added. The citric acid is next added and mixed well. The remaining ingredients are then added and the mixture poured on a kneading table, kneaded approximately seven minutes and lozenges then formed.

EXAMPLE 2

| Ingredient | Amount/Lozenge |
|---|---|
| 3-Piperidino-p-butoxypropiophenone HCl | 1.20 mg |
| Anhydrous Citric Acid | 20.40 mg |
| Flavouring | 12.42 mg |
| Colouring Agent | 0.222 mg |
| Anhydrous Candy Base to | QS 2.40 gm |

EXAMPLE 3

| Ingredient | Amount/Lozenge |
|---|---|
| 3-Piperidino-p-butoxypropiophenone HCl | 2.40 mg |
| Anhydrous Citric Acid | 20.40 mg |
| Flavouring | 12.42 mg |
| Colouring Agent | 0.222 mg |
| Anhydrous Candy Base to | QS 2.40 gm |

EXAMPLE 4

| Ingredient | Amount/Lozenge |
|---|---|
| 3-Piperidino-*p*-butoxypropiophenone HCl | 24.0 mg |
| Menthol USP | 6.62 mg |
| Colouring (green) | 0.222 mg |
| Flavouring | 5.8 mg |
| Anhydrous Citric Acid | 20.40 mg |
| Anhydrous Candy Base to | QS 2.4 gm |

**Claims**

1. A method of preparing topical anaesthetic lozenges containing a pharmaceutically acceptable acid addition salt of a compound of formula (I)

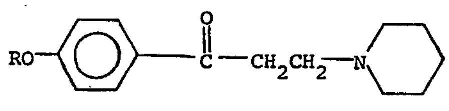

(I)

wherein R is alkyl of 2 to 8 carbon atoms by incorporating said acid addition salt in a molten candy base and moulding into lozenges, characterised in that citric acid, fumaric acid, malic acid, tartaric acid, lactic acid, adipic acid or phosphoric acid is added to the molten candy base prior to or simultaneously with the addition of said acid addition salt, in an amount to constitute from 0.2% to 1% by weight of the resultant lozenges.

2. A method as claimed in claim 1 characterised in that citric acid is added in an amount to constitute from 0.25% to 0.5% of the resultant lozenges.

3. A method as claimed in claim 1 or claim 2 characterised in that said acid addition salt is the hydrochloride salt.

4. A method as claimed in any one of claims 1 to 3 characterised in that said acid addition salt is of a compound of formula (I) wherein R is n-butyl.

5. A topical anaesthetic lozenge comprising a pharmaceutically acceptable acid addition salt of a compound of formula (I)

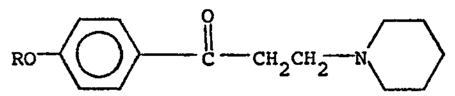

(I)

wherein R is alkyl of from 2 to 8 carbon atoms in a hard candy base, characterised in that citric acid, fumaric acid, malic acid, tartaric acid, lactic acid, adipic acid or phosphoric acid is also present, in an amount from 0.2% to 1.0% by weight of each lozenge.

6. A topical anaesthetic lozenge according to claim 5 characterised in that citric acid is present in an amount from 0.25% to 0.5% by weight of each lozenge.

7. A topical anaesthetic lozenge according to claim 5 or claim 6 characterized in that said acid addition salt is the hydrochloride salt.

8. A topical anaesthetic lozenge according to any one of claims 5 to 7 characterised in that said acid addition salt is of a compound of formula (I) wherein R is n-butyl.

**Revendications**

1. Procédé pour la préparation de pastilles ou tablettes à effet anesthésique local contenant un sel d'addition avec un acide pharmaceutiquement acceptable d'un composé de formule (I)

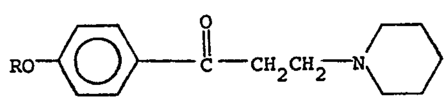

(I)

dans laquelle R est un alcoyle ayant de 2 à 8 atomes de carbone, par l'incorporation d'un sel d'addition avec un acide à une base de sucre candi fondu et par moulage en pastilles ou tablettes, caractérisé en ce qu'on ajoute de l'acide citrique de l'acide fumarique, de l'acide maléique, de l'acide tartrique, de l'acide lactique de l'acide adipique ou de l'acide phosphorique à la base de sucre candi fondu avant ou simultanèment à l'addition du sel d'addition avec un acide, en une quantité représentant de 0,2% à 1% en poids des pastilles ou tablettes résultantes.

2. Procédé suivant la revendication 1, caractérisé en ce que de l'acide citrique est ajouté en une quantité représentant de 0,25% à 0,5% des pastilles ou tablettes résultantes.

3. Procédé suivant la revendication 1 ou 2, caractérisé en ce que ce sel d'addition avec un acide est le chlorhydrate.

4. Procédé suivant l'une quelconque des revendications 1 à 3, caractérisé en ce que le sel d'addition avec un acide est celui d'un composé de formule (I) dans laquelle R est un n-butyle.

5. Pastille ou tablette à effet anesthésique local, comprenant un sel d'addition avec un acide pharmaceutique acceptable d'un composé de formule (I).

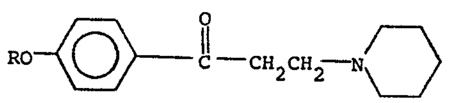

(I)

dans laquelle R est un alcoyle ayant de 2 à 8 atomes de carbone, dans une base de sucre candi dur, caractérisée en ce que de l'acide citrique, de l'acide fumarique, de l'acide maléïque, de l'acide tartrique, de l'acide lactique de l'acide adipique ou de l'acide phosphorique est également présent en une quantité allant de 0,2% à 1,0% en poids de chaque pastille ou tablette.

6. Pastille ou tablette à effet anesthésique local suivant la revendication 5, caractérisée en ce que l'acide citrique est présent en une quantité allant de 0,25% à 0,5% en poids de chaque pastille ou tablette.

7. Pastille ou tablette à effet anesthésique local suivant la revendication 5 ou 6, caractérisée en ce que ce sel d'addition avec un acide est le chlorhydrate.

8. Pastille ou tablette à effet anesthésique local suivant l'une quelconque des revendications 5 à 7, caractérisée en ce que ce sel d'addition avec un acide est un composé de formule (I) dans laquelle R est un n-butyle.

**Patentansprüche**

1. Verfahren zur Herstellung von lokalanästhetischen Lutschbonbons, enthaltend ein pharmakologisch verträgliches Säureadditionalssalz einer Verbindung der Formel I

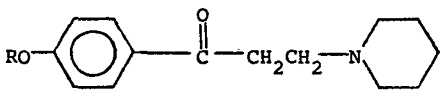

(I)

in der R ein Alkylrest mit 2 bis 8 Kohlenstoffatomen ist, durch Einverleiben des Säureadditionssalzes in eine geschmolzene Zuckermasse und Formen zu Lutschbonbons, dadurch gekennzeichnet, daß Citroenensäure, Fumarsäure, Malonsäure, Weinsäure, Milchsäure, Adipinsäure oder Phosphorsäure vor oder gleichzeitig mit der Zugabe des Säureadditionssalzes in einer solchen Menge zu der geschmolzenen Zuckermasse zugesetzt wird, daß sie 0,2 bis 1 Gewichtsprozent der entstehenden Lutschbonbons ausmacht.

2. Verfahren nach Anspruch 1, dadurch gekennzeichnet, daß Citronensäure in einer solchen Menge zugesetzt wird, daß sie 0,25 bis 0,5 Gewichtsprozent der entstehenden Lutschbonbons ausmacht.

3. Verfahren nach Anspruch 1 oder 2, dadurch gekennzeichnet, daß das Säureadditionssalz das Hydrochlorid ist.

4. Verfahren nach Anspruch 1 bis 3, dadurch gekennzeichnet, daß das Säureadditionssalz das Salz einer Verbindung der Formel I ist, in der R ein n-Butylrest ist.

5. Lokalanästhetisches Lutschbonbon, enthaltend ein pharmakologisch verträgliches Säureadditionssalze einer Verbindung der Formel I

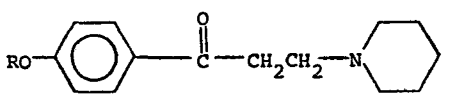

(I)

in der R ein Alkylrest mit 2 bis 8 Kohlenstoffatomen ist, in einer harten Zuckermasse, dadurch gekennzeichnet, daß Citronensäure, Fumarsäure, Malonsäure, Weinsäure, Milchsäure, Adipinsäure oder Phosphorsäure auch vorhanden ist in einer Menge von 0,2 bis 1,0 Gewichtsprozent je Lutschbonbon.

6. Lokalanästhetisches Lutschbonbon nach Anspruch 5, dadurch gekennzeichnet, daß Citronensäure in einer Menge von 0,25 bis 0,5 Gewichtsprozent je Lutschbonbon vorhanden ist.

7. Lokalanästhetisches Lutschbonbon nach Anspruch 5 oder 6, dadurch gekennzeichnet, daß das Säureadditionssalz das Hydrochlorid ist.

8 Lokalanästhetisches Lutschbonbon nach einem der Ansprüche 5 bis 7, dadurch gekennzeichnet, daß das Säureadditionssalz das Salz einer Verbindung der Formel I ist, in der R ein n-Butylrest ist.